# FASCICULE DE BREVET EUROPEEN

(11) **EP 4 139 675 B1**
(45) Date de publication et mention de la délivrance du brevet: **05.06.2024**
(21) Numéro de dépôt: 21720581.4
(22) Date de dépôt: 20.04.2021
(51) Int. Cl.: G01N 33/18, F03B 17/06, C02F 1/00, C02F 1/32, C02F 1/46, C02F 1/66, C02F 103/42

(54) **DISPOSITIF D'ANALYSE D'EAU AUTONOME ET PISCINE EQUIPÉE D'UN TEL DISPOSITIF**
AUTONOME WASSERANALYSEVORRICHTUNG UND SCHWIMMBAD MIT SOLCH EINER VORRICHTUNG
AUTONOMOUS WATER ANALYSIS DEVICE AND SWIMMING POOL EQUIPPED WITH SUCH A DEVICE

(30) Priorité: 21.04.2020 FR 2003981
(43) Date de publication de la demande: 01.03.2023
(73) Titulaire: Bleu Electrique, 13016 Marseille (FR)
(72) Inventeur: BARET, Emmanuel, 13007 Marseille (FR)
(74) Mandataire: Hautier IP
(86) Numéro de dépôt international: PCT/IB2021/053252
(87) Numéro de publication internationale: WO 2021/214656

(56) Documents cités:
- DE-U1-202009 002 145
- US-A1- 2010 250 449
- US-A1- 2018 297 862
- HOFFMANN D ET AL: "Energy Harvesting from Fluid Flow in Water Pipelines for Smart Metering Applications", JOURNAL OF PHYSICS: CONFERENCE SERIES, INSTITUTE OF PHYSICS PUBLISHING, BRISTOL, GB, vol. 476, no. 1, 4 December 2013 (2013-12-04), page 12104, XP020254816, ISSN: 1742-6596, DOI: 10.1088/1742-6596/476/1/012104 [retrieved on 2013-12-04]

## Description

### Domaine technique

La présente invention concerne un dispositif d'analyse d'eau autonome, utilisable en particulier pour l'analyse de l'eau de piscines. Plus particulièrement, le dispositif de la présente invention est un dispositif qui vise à mesurer et transmettre des valeurs physico-chimiques de l'eau, tels que la température, la turbidité, le débit, la pression, le pH ou analogue. Elle concerne également une piscine équipée d'un tel dispositif.

L'invention trouve des applications dans le contrôle de paramètres physico-chimiques de l'eau d'une piscine et la surveillance de la qualité et de la sécurité sanitaire de l'eau.

L'invention trouve également des applications dans la commande des organes d'un circuit de filtration d'une piscine.

### Etat de la technique antérieure

La garantie de la qualité de l'eau d'une piscine nécessite la surveillance d'un certain nombre de paramètres physico-chimiques, tels que la température de l'eau, le potentiel-hydrogène (pH), le potentiel d'oxydo-réduction (ORP), la salinité (S) de l'eau, le taux de solides dissous (TDS), et la turbidité de l'eau.

Ces paramètres peuvent être pris en compte pour piloter un circuit de filtration de la piscine ou pour commander un certain nombre d'équipements d'un local technique de la piscine, tels que les pompes doseuses, prévues pour déverser dans l'eau du circuit de filtration des additifs correcteurs. Il s'agit par exemple de produits de traitement comme du chlore, du brome, de l'oxygène actif, ou un liquide de correction de potentiel-hydrogène.

Les dispositifs d'analyse de l'eau peuvent être installés dans le local technique de la piscine et mesurer les paramètres de l'eau traversant une canalisation ou une dérivation du circuit de filtration de la piscine. Ils peuvent être alimentés en énergie par un réseau de distribution électrique ou par un accumulateur électrique, rechargeable ou non.

On connait également des dispositifs d'analyse et de contrôle configurés pour flotter sur l'eau de la piscine. Ces dispositifs sont généralement pourvus d'une batterie rechargeable pour leur alimentation électrique ainsi que d'un émetteur-récepteur pour la transmission des données d'analyse vers les équipements du local technique.

Le document US 2018/0297862 décrit un système de traitement d'eau d'une piscine par électrolyse pour désinfecter l'eau. Plus particulièrement, le système a pour but de réguler la production d'un désinfectant. Le système comprend une enceinte cylindrique ménagée autour d'un axe de révolution. L'enceinte cylindrique loge un dispositif de traitement électrolytique, un dispositif de contrôle électronique et un dispositif de génération hydraulique.

L'enceinte cylindrique s'étend axialement entre une première extrémité pourvue d'une entrée d'eau et une deuxième extrémité munie d'une sortie d'eau. A l'intérieur de l'enceinte cylindrique, l'eau circule entre l'entrée d'eau et la sortie d'eau selon un sens d'écoulement qui est parallèle à l'axe de révolution. Il en découle que le dispositif de traitement électrolytique, le dispositif de contrôle électronique et le dispositif de génération hydraulique baignent totalement dans l'eau, ce qui présente un risque au regard de l'étanchéité nécessaire au dispositif de contrôle électronique et/ou au dispositif de génération hydraulique.

Le dispositif de génération hydraulique comprend une turbine destinée à produire l'énergie électrique nécessaire à l'électrolyse de l'eau. Mais le dimensionnement de cette turbine, mobile en rotation autour d'un axe de rotation qui est parallèle à l'axe de révolution et au sens d'écoulement de l'eau à l'intérieur de l'enceinte cylindrique, ne permet pas de générer l'énergie suffisante pour électrolyser de l'eau salée dans les conditions décrites. Il en découle une inaptitude à remplir sa fonction.

### Exposé de l'invention

L'invention procède de la mise en évidence d'un certain nombre de difficultés rencontrées avec les dispositifs d'analyse d'eau connus.

Pour les dispositifs d'analyse d'eau alimentés par un réseau électrique, une difficulté tient à leur connexion au réseau et à la nécessité de garantir la parfaite isolation électrique de la connexion par rapport au milieu liquide.

Pour les dispositifs autonomes d'analyse d'eau comprenant un dispositif de génération électrique associant une turbine et une batterie, il est également souhaité de garantir de manière pérenne une étanchéité totale au regard de composants électriques et/ou électroniques constitutifs du dispositif de génération électrique et/ou de la batterie, vis-à-vis de l'eau circulant à l'intérieur du dispositif autonome d'analyse d'eau.

Une difficulté rencontrée avec les dispositifs d'analyse à batterie est celle de la pérennité de leur fonctionnement. En effet, si la batterie vient à être déchargée, un contrôle de la qualité sanitaire de l'eau de la piscine devient aléatoire ou impossible. En outre, la charge de la batterie nécessite également une connexion électrique.

Il en découle un besoin de disposer d'un dispositif autonome d'analyse d'eau comprenant un dispositif de génération électrique performant et efficace pour charger efficacement la batterie.

Encore une autre difficulté mise en évidence est une influence d'un éventuel défaut du système de filtration, sur l'analyse et le contrôle des paramètres de l'eau. Un défaut du système de filtration peut résulter, par exemple, d'un colmatage d'un filtre, un encrassement d'une pompe, la fermeture inopinée d'une vanne ou une obstruction de l'écumeur (skimmer). Un tel défaut peut fausser la mesure des paramètres de l'eau et empêcher une mise en oeuvre d'actions correctrices adaptées.

La présente invention a pour but de proposer un dispositif d'analyse de l'eau, et notamment de l'eau d'une piscine, qui permette d'obvier à ces difficultés.

Un but est en particulier de proposer un tel dispositif d'analyse qui soit parfaitement autonome, sans aucun fil ou raccordement électrique, et qui ne nécessite aucune intervention humaine pour garantir la continuité de son alimentation électrique.

Un autre but est de proposer un tel dispositif d'analyse permettant de prendre en compte et de signaler au besoin un défaut du système de filtration d'une piscine qui en est équipée.

Un autre but est de proposer un tel dispositif d'analyse qui est simple et compact, notamment exempt d'un dispositif de traitement de l'eau, mais qui a pour fonction de mesurer et transmettre des valeurs de paramètres physico-chimiques de l'eau, tels que la température, la turbidité, le débit, la pression, le pH ou analogue, à un récepteur en vue d'actionner un autre système, distinct du dispositif d'analyse.

L'invention a également pour but de proposer une piscine équipée d'un tel dispositif d'analyse.

Pour atteindre ces buts, l'invention propose un dispositif d'analyse d'eau selon la revendication 1.

La chambre d'analyse comporte une entrée d'eau et une sortie d'eau, pourvues au besoin de raccords pour connecter la chambre d'analyse à des conduites d'un circuit d'eau. Par ailleurs, la chambre d'analyse peut être configurée pour réduire le débit d'un courant d'eau qui la traverse, notamment au voisinage de la sonde ou des sondes. Un débit réduit au voisinage de la sonde permet de garantir une meilleure fiabilité des mesures en évitant des perturbations susceptibles d'être occasionnées par un mouvement rapide de l'eau, telle une cavitation ou la génération d'électricité statique. La réduction du débit est locale au voisinage des sondes, elle est sans effet sur le débit d'eau global traversant la chambre d'analyse.

Le boîtier étanche est adjacent à la chambre d'analyse et de préférence solidaire de la chambre d'analyse. Le boîtier étanche peut être formé d'une seule pièce avec la chambre d'analyse avec une cloison étanche de séparation.

Le boîtier étanche surplombe la chambre d'analyse et est contigu à la chambre d'analyse. Autrement dit, la chambre d'analyse et le boîtier étanche sont ménagés côte-à-côte l'un de l'autre en étant séparés l'un de l'autre par une cloison commune à l'un et à l'autre.

L'étanchéité du boîtier étanche, s'entend principalement par rapport à la chambre d'analyse de sorte que de l'eau qui emplit la chambre d'analyse ou qui traverse la chambre d'analyse ne puisse atteindre les composants logés dans le boîtier étanche. Le boîtier étanche peut être étanche non seulement par rapport à la chambre d'analyse mais aussi, et plus généralement, par rapport à l'extérieur du boîtier. L'étanchéité permet, dans ce cas, de mettre les composants logés dans le boîtier à l'abri de l'eau et de l'humidité ambiante.

La ou les sondes du dispositif peuvent être disposées entièrement, ou en partie, dans la chambre d'analyse pour garantir la mise en contact physique des sondes avec l'eau emplissant la chambre d'analyse ou circulant à travers la chambre d'analyse.

La sonde, ou les sondes, peuvent être choisies parmi une sonde de température, une sonde de pH, une sonde de potentiel d'oxydo-réduction, une sonde de salinité, une sonde de taux de solides dissous, une sonde ampérométrique et une sonde de turbidité, par exemple.

Le terme « sonde » est compris comme tout composant ou capteur sensible à une propriété chimique ou physique de l'eau, ou du flux d'eau (pression, débit, vitesse), et capable de délivrer un signal susceptible d'être appliqué à l'entrée de données du processeur de données. Le signal peut être appliqué directement à l'entrée du processeur de données dans le cas d'une sonde numérique, ou peut y être appliqué par l'intermédiaire d'une interface, comprenant, par exemple un ou plusieurs convertisseurs analogique-numérique et/ou étages analogiques de mise en forme des signaux (amplificateur ou autre système de conditionnement).

Le générateur électrique du dispositif d'analyse est alimenté en énergie par la turbine dont le mouvement est provoqué par un flux d'eau traversant la chambre d'analyse. On entend par générateur électrique tout dispositif permettant de convertir l'énergie mécanique de la turbine en énergie électrique. Le générateur électrique peut comporter, par exemple, l'un parmi une dynamo et un alternateur. L'énergie électrique fournie par le générateur électrique permet d'alimenter le processeur de données, les sondes, et plus généralement l'ensemble des composants électriques ou électroniques logés dans le boîtier étanche.

La turbine comporte une hélice ou des pales disposées dans la chambre d'analyse ou une partie de la chambre d'analyse susceptible d'être traversée par un flux d'eau.

La turbine peut également être pourvue d'un arbre moteur reliant son hélice ou ses pales à la dynamo ou à l'alternateur du générateur électrique, logés dans le boîtier étanche.

Toutefois, pour éviter qu'un arbre moteur ne traverse une cloison entre la chambre d'analyse et le boîtier étanche, le générateur électrique peut comporter un inducteur à aimants permanents, solidaire de la turbine, et un induit logé dans le boîtier étanche. Dans ce cas, aucun arbre ou organe mobile ne traverse la cloison et tout risque d'un défaut d'étanchéité à l'eau peut être évité. L'énergie électrique est produite par un couplage magnétique entre l'inducteur et l'induit à travers la cloison qui sépare la chambre d'analyse et le boîtier étanche.

Selon un perfectionnement, l'alimentation électrique peut comporter en outre un accumulateur d'énergie électrique logé à l'intérieur du boîtier étanche. Il s'agit, par exemple, d'une batterie rechargeable.

Le générateur électrique peut également être relié à l'accumulateur d'énergie, c'est à dire à la batterie, de manière à la charger et maintenir sa charge.

La batterie a essentiellement pour fonction de garantir la continuité du fonctionnement du dispositif d'analyse, y compris en l'absence temporaire de circulation d'eau à travers la chambre d'analyse.

En l'absence de flux d'eau, la turbine n'est pas entrainée et le générateur électrique délivre une tension et un courant nuls. La tension nulle appliquée à l'entrée de données du processeur de données peut alors être utilisée comme un signal informant le processeur de données d'un éventuel dysfonctionnement d'un circuit de filtration d'une piscine équipée du dispositif d'analyse. Il s'agit, par exemple, d'une panne d'une pompe de circulation ou un colmatage d'un filtre, qui empêche l'eau de circuler. Le processeur de données peut alors piloter l'émission d'un signal d'alerte ou une commande de déclenchement d'un signal d'alerte. Le processeur de données peut également piloter une inhibition d'éventuels équipements correcteurs de l'eau. Ceci permet d'éviter par exemple, qu'un agent chimique correcteur de l'eau ne soit déversé dans une conduite du circuit de filtration dans laquelle l'eau ne circule pas.

Il convient de préciser que la pompe de circulation n'est pas nécessairement en fonctionnement de manière permanente. Ainsi, il est envisageable de n'émettre une alerte que lorsqu'un dysfonctionnement de la circulation de l'eau du circuit de filtration est détecté alors que la pompe est sous tension au moment de la détection du dysfonctionnement. La mise sous tension de la pompe ou sa mise à l'arrêt peuvent être des informations transmises au processeur de données par voie hertzienne de la manière décrite plus loin. Une alerte peut également être émise lorsqu'un dysfonctionnement de la circulation de l'eau, c'est-à-dire l'absence de rotation de la turbine, est détecté pendant une certaine durée de temps configurable, par exemple, une durée de vingt-quatre heures.

Selon une possibilité avantageuse de mise en oeuvre de l'invention, le signal fonction de la vitesse de rotation de la turbine peut être une tension de sortie du générateur électrique. Comme la tension délivrée par le générateur électrique est fonction de la vitesse de rotation de la turbine elle est également fonction du débit de l'eau à travers la chambre d'analyse. Ce signal est alors appliqué à l'entrée de données du processeur de données. Lorsque la chambre d'analyse reçoit l'eau d'un circuit de filtration d'une piscine, la tension du générateur est également fonction de la quantité d'eau traitée par unité de temps. La turbine et le générateur électrique sont ainsi utilisés comme un capteur de débit d'eau.

De manière générale, la tension délivrée par un générateur électrique, que ce soit un alternateur, une dynamo ou une autre machine tournante comparable, est fonction de la vitesse de rotation de l'arbre. En première approximation il s'agit d'une fonction de proportionnalité.

Il convient de préciser qu'au lieu de la tension délivrée par le générateur, il est également possible de mesurer un courant délivré par le générateur, ou un courant de dérivation proportionnel, en appliquant un signal proportionnel au courant ou à la tension du générateur électrique à l'entrée de mesure du processeur de données. Dans la présente description, la mention de la tension du générateur est ainsi comprise comme n'excluant pas l'utilisation éventuelle d'une autre grandeur proportionnelle à la vitesse de rotation de la turbine comme signal d'entrée du processeur de données. A titre d'exemple, le générateur électrique peut être équipé d'un compte tours par détection d'aimant ou de tout autre système permettant de détecter la vitesse de rotation de la turbine, et permettant de générer un signal à destination du processeur de données.

Le processeur de données peut être un processeur numérique tel qu'un microprocesseur ou un microcontrôleur. Selon un mode de réalisation avantageux du dispositif d'analyse, le processeur de données peut être équipé d'un émetteur-récepteur hertzien logé dans le boîtier étanche et configuré pour la transmission d'au moins l'un parmi des données d'analyse, des données de commande et des données d'alerte.

Grâce à l'émetteur-récepteur, les données sont transmises par voie hertzienne. On considère que des données sont transmises par voie hertzienne lorsqu'elles sont transmises sans fil, par des ondes électromagnétiques, et ce sans préjuger de la fréquence des ondes. L'émetteur-récepteur, est, par exemple un émetteur-récepteur radiofréquence. Des données peuvent être transmises depuis le processeur de données vers des équipements extérieurs. Des données peuvent également être reçues par le processeur de données depuis des équipements extérieurs.

On entend par « données d'analyse » des données représentatives de propriétés physico-chimiques de l'eau ou représentatives de la qualité sanitaire de l'eau. Ces données peuvent inclure des valeurs de température, de débit, de pression, de pH (potentiel-hydrogène), d'ORP (potentiel d'oxydo-réduction), de salinité, de turbidité, etc.

On entend par « données de commande » des données élaborées par le processeur de données et susceptibles d'être reçues par des équipements extérieurs au dispositif d'analyse pour exécuter des opérations en lien avec l'entretien de la qualité de l'eau d'une piscine. Ces équipements peuvent comporter une unité de traitement de l'eau par rayonnement ultraviolet, un système d'électrolyse au sel, des pompes doseuses pour injecter dans l'eau d'une piscine des additifs correcteurs, mais aussi des pompes de circulation ou des vannes. L'ensemble de ces équipements peuvent être contrôlés et pilotés en fonction des paramètres physico-chimiques de l'eau mesurés par les sondes mais aussi en fonction du débit d'un circuit de filtration établi à partir de la vitesse de rotation de la turbine et/ou la tension délivrée par le générateur électrique.

On entend par « données d'alerte » des données utilisables pour déclencher un système d'alerte, lumineux ou sonore, ou encore des données susceptibles d'être émises sous la forme de messages vers un ou plusieurs appareils de communication distants. Les données d'alerte sont émises lorsque le processeur constate une dérive d'une ou de plusieurs données d'analyse en dehors de plages de consigne prédéfinies et mémorisées. Plusieurs modes de communication hertzienne entre le dispositif d'analyse et des équipements extérieurs peuvent être prévus. Une communication peut être établie par liaison radiofréquence avec des équipements d'un local technique d'une piscine comprenant les pompes, les appareils de traitement ou de chauffage, les vannes ou les pompes doseuses précitées. Dans ce cas, le processeur de données peut émettre des commandes ou des valeurs pour le pilotage de ces équipements. Outre les données d'analyse et les données de commande, le processeur peut aussi émettre des données d'information relatives à l'état du dispositif d'analyse. Il s'agit, par exemple, d'informations relatives au fonctionnement du processeur de données ou à l'état de charge de la batterie.

Le dispositif d'analyse peut également être équipé d'un module de communication Wifi, Bluetooth ou un module de communication par réseau étendu à basse consommation (LPWAN). Ceci permet d'établir une communication entre le processeur de données et un équipement tel qu'un ordinateur personnel, une tablette, ou un smartphone, ou un objet connecté pour l'affichage des données d'analyse. Le dispositif d'analyse peut également utiliser cette communication pour envoyer ses données de fonctionnement et les valeurs mesurées sur un serveur Internet, permettant ensuite la restitution d'un historique des valeurs mesurées. Enfin, le réseau de communication peut servir à envoyer des alertes à un utilisateur du système, et ce même si l'utilisateur n'utilise pas son ordinateur personnel, sa tablette, ou son smartphone lors de l'apparition d'une alerte. Une alerte peut être émise par envoi d'une notification sur un appareil de l'utilisateur, par l'envoi de SMS, par l'envoi d'email, ou de tout autre type de message.

Selon une autre possibilité, non exclusive de la précédente, le processeur de données peut également être équipé d'une interface de sortie à commutateurs. Une telle interface, conçue pour une éventuelle connexion filaire d'équipements, peut comporter, par exemple, un ou plusieurs relais électromécaniques ou statiques, dont l'état de commutation est piloté par le processeur de données.

Selon encore une autre possibilité, non exclusive de la précédente, le processeur de données peut être équipé d'une interface d'entrée conçue pour une connexion filaire d'une sonde ou d'un capteur externe capable de délivrer un signal appliqué à l'entrée du processeur de données pour s'ajouter aux « données d'analyse » et être éventuellement transmises par voie hertzienne vers des équipements extérieurs.

L'invention concerne également une piscine comprenant un circuit de filtration pourvu d'une pompe de circulation, et d'au moins un filtre. Conformément à l'invention le circuit de filtration est en outre pourvu d'un dispositif d'analyse tel que décrit précédemment.

Le terme « piscine » est compris comme englobant les piscines privées ou publiques mais aussi les bains à remous.

Le dispositif d'analyse met à profit le flux d'eau provoqué par la pompe de circulation dans le circuit de filtration pour entretenir le mouvement de la turbine et générer l'énergie nécessaire au fonctionnement de la turbine.

La mise en place d'un dispositif d'analyse dans le circuit de filtration existant d'une piscine ne nécessite aucune connexion électrique mais simplement un raccordement fluidique, de manière que tout ou partie de l'eau du circuit de filtration traverse la chambre d'analyse.

De préférence, le dispositif d'analyse peut être connecté en aval du filtre. Cette mesure permet d'éviter que d'éventuelles particules, susceptibles d'être retenues par le filtre, ne viennent obturer la chambre d'analyse, ou ne viennent entraver le mouvement de la turbine.

Par ailleurs, le dispositif d'analyse peut avantageusement être connecté en série avec la pompe de circulation et le filtre, une entrée d'eau et une sortie d'eau de la chambre d'analyse étant reliés à une conduite du circuit de filtration.

Dans ce cas, le dispositif d'analyse constitue également un témoin du bon fonctionnement du circuit de filtration. Un éventuel défaut de la pompe de circulation ou un colmatage du filtre se traduisent par une diminution ou une interruption du flux d'eau à travers la chambre d'analyse et donc une diminution ou une annulation de la vitesse de rotation de la turbine.

La vitesse de rotation de la turbine est établie et surveillée par le processeur de données recevant à son entrée de données la tension du générateur électrique. Elle peut être comparée à une valeur ou une plage de consigne stockée dans une mémoire. La vitesse de rotation de la turbine, indicatrice du flux d'eau traversant la chambre d'analyse peut également être utilisée comme paramètre correcteur des autres grandeurs physico-chimiques établis pour l'analyse de l'eau.

Un branchement du dispositif d'analyse sur une dérivation du circuit de filtration est également envisageable.

Comme évoqué précédemment, la piscine peut comporter en outre au moins une pompe doseuse de liquide correcteur. La pompe doseuse peut être pilotée en fonction d'au moins une donnée d'analyse élaborée par le processeur de données.

La pompe doseuse peut être pilotée soit directement par le processeur de données, soit par l'intermédiaire d'une unité de pilotage externe au dispositif d'analyse, recevant la donnée d'analyse élaborée par le processeur de données et fixant une dose de liquide correcteur en fonction de cette donnée. L'unité de pilotage est, par exemple, une alimentation électrique de la pompe doseuse en communication hertzienne avec le dispositif d'analyse.

D'autres caractéristiques et avantages de l'invention ressortent de la description qui suit en référence aux figures des dessins. Cette description est donnée à titre illustratif et non limitatif.

### Brève description des figures

La figure 1 est une représentation schématique simplifiée d'un dispositif d'analyse conforme à l'invention.
La figure 2 est une vue en perspective du dispositif d'analyse illustré sur la figure 1.
La figure 3 est une vue éclatée du dispositif d'analyse illustrée sur les figures 1 et 2.
La figure 4 est une autre vue éclatée du dispositif d'analyse illustrée sur les figures 1 à 3.
La figure 5 est une représentation schématique simplifiée d'une piscine pourvue d'un circuit de filtration et d'un dispositif d'analyse conforme à l'invention.

Les figures sont représentées en échelle libre.

### Description détaillée de modes de mise en oeuvre de l'invention

Dans la description qui suit, des parties identiques similaires ou équivalentes sont repérées avec les mêmes signes de référence de manière à pouvoir se reporter d'une figure à l'autre.

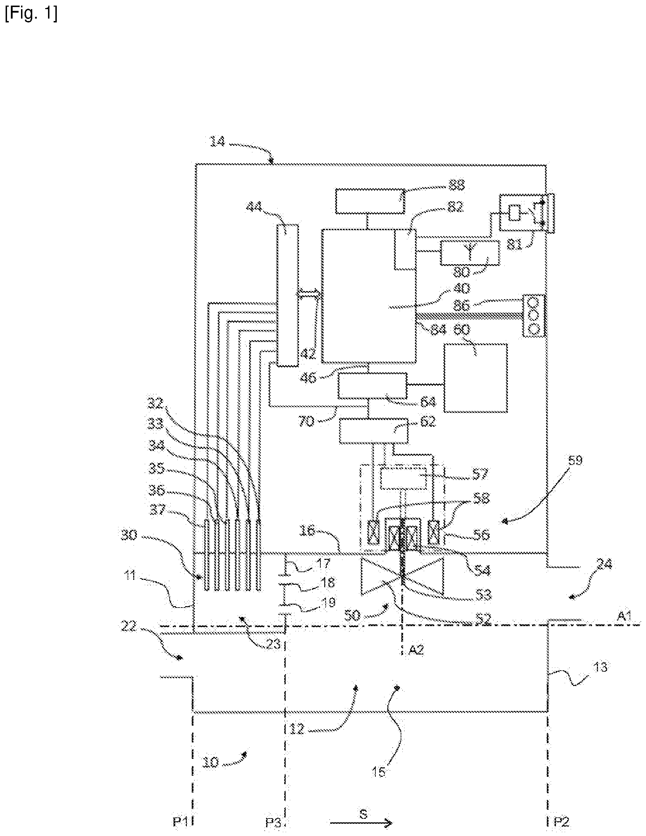

La figure 1 montre, de manière schématique, un dispositif d'analyse 10 conforme à l'invention. Il comprend une chambre d'analyse 12, vue en coupe, et un boîtier étanche 14 logeant des composants électriques et électroniques. La chambre d'analyse 12 est séparée du boîtier étanche 14 par une cloison 16.

La chambre d'analyse 12 est globalement conformée en un cylindre d'axe de révolution A1 qui forme un axe d'extension générale de la chambre d'analyse 12. Le boîtier étanche 14 surplombe la chambre d'analyse 12. Autrement dit, les composants électriques et électroniques du dispositif d'analyse 10 sont rassemblés à l'intérieur d'un unique boîtier étanche 14 qui jouxte la chambre d'analyse 12. Autrement dit encore, la chambre d'analyse 12 et le boîtier étanche 14 sont contigus l'un à l'autre en étant séparés l'un de l'autre par une unique cloison 16.

La chambre d'analyse 12 présente une entrée d'eau 22 et une sortie d'eau 24 susceptibles d'être connectées à une conduite d'eau.

L'entrée d'eau 22 est ménagée à travers une première paroi 11 délimitant la chambre d'analyse 12 et la sortie d'eau 24 est ménagée à travers une deuxième paroi 13 délimitant la chambre d'analyse 12. La première paroi 11 et la deuxième paroi 13 sont disposées en vis-à-vis l'une de l'autre et sont sécantes avec l'axe de révolution A1. Plus particulièrement, la première paroi 11 s'étend à l'intérieur d'un premier plan P1 et la deuxième paroi 13 s'étend à l'intérieur d'un deuxième plan P2, le premier plan P1 et le deuxième plan P2 étant sensiblement orthogonaux à l'axe de révolution A1. Ces dispositions sont telles que la chambre d'analyse 12 est susceptible d'être parcourue par un fluide 15, eau notamment ou analogue, selon un sens d'écoulement S du fluide 15 qui est sensiblement parallèle à l'axe de révolution A1. On note que selon une variante de réalisation, l'entrée d'eau 22 est susceptible d'occuper toute la surface de la première paroi 11 offerte dans le premier plan P1 et que la sortie d'eau 22 est susceptible d'occuper toute la surface de la deuxième paroi 13 offerte dans le premier plan P2.

Un jeu de sondes 30, s'étendent dans la chambre d'analyse 12. Le jeu de sondes 30 comprend une sonde de température 32, une sonde de pH 33, une sonde de potentiel d'oxydo-réduction 34, et une sonde de salinité 35. Le jeu de sondes 30 peut être complété par une sonde de solides dissous 36 et une sonde de turbidité 37.

On peut noter que les sondes 30 sont logées dans un compartiment 23 de la chambre d'analyse 12, en communication avec la chambre d'analyse 12. Le compartiment 23 a pour fonction de réduire le débit de l'eau traversant la chambre d'analyse 12 au voisinage des sondes 30, pour éviter qu'un débit trop fort ne risque de perturber les mesures.

A cet effet, le compartiment 23 est par exemple interposé radialement entre l'entrée d'eau 21 et la cloison 16 qui délimite la chambre d'analyse 12 et le boîtier étanche 14. Selon une forme de réalisation, le compartiment 23 est délimité par la première paroi 11, la cloison 16 et au moins une troisième paroi 17. La troisième paroi 17 comporte une bouche d'admission d'eau 18 et une bouche d'évacuation d'eau 19 à travers lesquelles l'eau 15 est à même de circuler. La troisième paroi 17 s'étend au moins partiellement à l'intérieur d'un troisième plan P3 qui est interposé entre le premier plan P1 et le deuxième plan P2 et qui est aussi orthogonal à l'axe de révolution A1.

L'ensemble des sondes du jeu de sondes 30 est relié à une entrée de données 42 d'un processeur de données 40. Elles y sont reliées par l'intermédiaire d'une interface 44 configurée pour conditionner le signal analogique et/ou convertir le signal analogique des sondes 30 en un signal numérique compatible avec l'entrée de données 42 du processeur de données 40. L'interface 44 permet également d'alimenter en énergie électrique d'éventuelles sondes actives telles que des sondes de mesure de conductivité ou de turbidité.

Dans la chambre d'analyse 12 se trouve également une turbine 50, pourvue d'une hélice 52, et entrainée en rotation par un flux d'eau 15 susceptible de circuler à travers la chambre d'analyse 12 depuis son entrée d'eau 22 vers sa sortie d'eau 24. L'hélice 52 est mobile sur elle-même en rotation autour d'un axe de rotation A2 qui est sensiblement orthogonal à l'axe de révolution A1, et donc au sens d'écoulement S de l'eau 15 entre l'entrée d'eau 22 et la sortie d'eau 24. Il en découle qu'une vitesse de rotation de l'hélice 52 est proportionnelle à une vitesse d'écoulement de l'eau 15 à l'intérieur de la chambre d'analyse 12 et donc précisément représentative de la vitesse d'écoulement de l'eau 15 à l'intérieur de la chambre d'analyse 12. On note que l'hélice 52 est disposée axialement à l'intérieur de la chambre d'analyse 12 entre le compartiment 23 et la sortie d'eau 24.

Ces dispositions sont telles que des pertes de charge affectant l'eau qui circule à l'intérieur de la chambre d'analyse sont avantageusement minimisées.

La turbine 50 est équipée d'aimants permanents qui forment l'inducteur 54 d'un générateur électrique 56. Les aimants permanents sont solidaires d'un rotor de la turbine 50 disposé dans la chambre d'analyse 12. Sur la figure 1, et par simplification, les aimants permanents sont représentés symboliquement sur un arbre 53 de la turbine 50, qui s'étend selon l'axe de rotation A2. Le générateur électrique 56 comprend également un induit 58 disposé à l'intérieur du boîtier étanche 14. L'induit 58 est formé, par exemple, par un ou plusieurs bobinages électriques. Ainsi, un stator de la turbine 50 est disposé dans le boîtier étanche 14 tandis que le rotor de la turbine 50 est immergé dans la chambre d'analyse 12. L'induit 58 et l'inducteur 54 du générateur électrique 56 sont magnétiquement couplés à travers la paroi étanche 16, et forment un alternateur 59. Ainsi, une rotation de la turbine 50 et donc de l'inducteur 54 provoque la circulation d'un courant électrique dans l'induit 58. Selon ce mode de réalisation, l'arbre 53 de la turbine 50 ne traverse pas la cloison 16 séparant la chambre d'analyse 12 du boîtier étanche 14, de sorte qu'aucun joint d'arbre tournant n'est nécessaire. Ces dispositions garantissent une étanchéité parfaite et pérenne entre la chambre d'analyse 12 et le boîtier étanche 14.

Selon une variante représentée en trait discontinu, l'alternateur 59 du générateur électrique 56 constitué essentiellement par l'inducteur 54 et l'induit 58, peut être remplacé par une dynamo 57 montée sur un prolongement de l'arbre 53 de la turbine 50. Dans ce mode de réalisation, l'arbre 53 de la turbine traverse la cloison 16 séparant la chambre d'analyse 12 du boîtier étanche 14. Un joint d'arbre, non représenté, est prévu.

Le générateur électrique 56 est relié à une entrée d'alimentation électrique du processeur de données 40 par l'intermédiaire d'un redresseur 62 et d'un convertisseur de tension 64 continu-continu (DC/DC), permettant de délivrer une tension d'alimentation adaptée au processeur de données 40.

Une batterie 60 est également reliée au convertisseur de tension 64 pour alimenter le processeur de données 40.

Un courant électrique peut circuler du générateur électrique 56 ou de la batterie 60 vers le processeur de données 40 lorsque le processeur de données 40 est alimenté par le générateur électrique 56, ou la batterie 60, respectivement. Un courant peut également circuler du générateur électrique 56 vers la batterie 60 pour charger la batterie 60.

Le courant et la tension d'alimentation du processeur de données 40 sont régulés par le convertisseur de tension 64. De même, le courant et la tension de charge de la batterie 60 sont régulés par le convertisseur de tension 64.

De la manière déjà indiquée, la batterie 60 permet de suppléer à une carence temporaire d'énergie produite par le générateur électrique 56, lorsque la turbine 50 n'est pas entraînée. Ceci permet de garantir la continuité du fonctionnement du processeur de données 40 sans qu'aucune connexion électrique vers l'extérieur du dispositif d'analyse 10 ne soit nécessaire, ni aucune intervention humaine comme un remplacement de pile ou un rechargement de batterie.

Sur la figure 1, il convient de noter une liaison électrique 70 qui relie le générateur 56 à l'entrée de données 42 du processeur de données 40. La liaison électrique 70 se fait par l'intermédiaire du redresseur 62 et par l'intermédiaire de l'interface 44 du processeur de données 40.

La tension redressée disponible à la sortie du redresseur 62 dépend de la tension délivrée par le générateur électrique 56. Elle est proportionnelle à la vitesse de rotation de la turbine 50 et donc au flux d'eau 15 qui traverse la chambre d'analyse 12. La prise en compte de cette tension par le processeur de données 40 permet au processeur de données 40 non seulement de surveiller le fonctionnement de la turbine 50, mais aussi de mesurer le flux d'eau traité, et de prendre en compte ces paramètres pour l'élaboration de commandes de gestion de l'eau de la piscine.

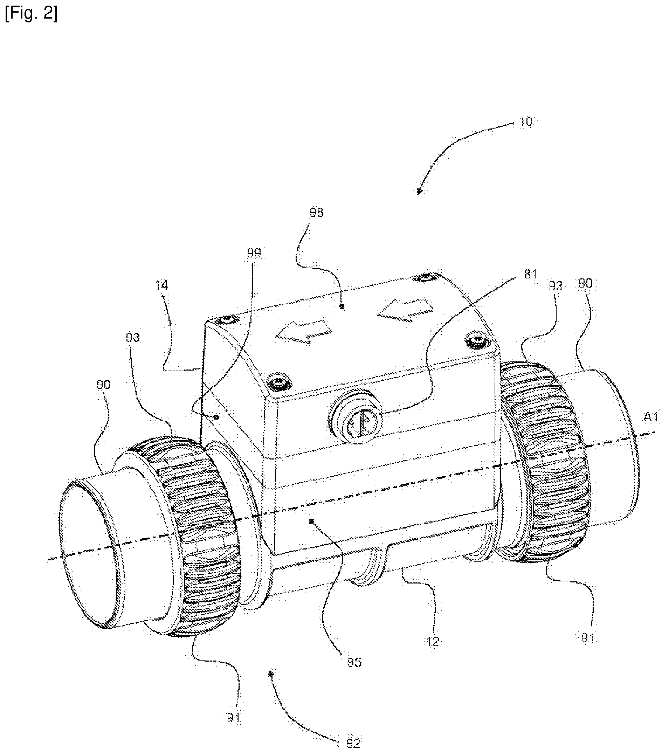

Parmi les données élaborées par le processeur de données 40, on peut distinguer les données d'analyse, les données d'alerte et les données de commande. Les données d'analyse, ou les données d'alerte, donnent un renseignement sur les propriétés physico-chimiques de l'eau ou la qualité sanitaire de l'eau qui traverse la chambre d'analyse 12. Elles peuvent être transmises à des équipements de surveillance ou d'affichage de la qualité de l'eau. Les données de commande sont des données destinées à des équipements gouvernant la gestion de l'eau de la piscine. Ces équipements, non représentés sur la figure 1, peuvent comporter, par exemple un chauffage de l'eau, une source de rayonnement UV, un système d'électrolyse au sel, des pompes doseuses d'agents correcteurs de l'eau de la piscine, une ou plusieurs pompes d'un circuit de filtration de l'eau de la piscine et une ou plusieurs électrovannes. De tels équipements sont décrits en référence à la figure 2.

Par retour à la figure 1, la transmission des données d'analyse, des données d'alerte et des données de commande, vers les équipements destinataires, a lieu par voie hertzienne. Ainsi, aucune liaison filaire n'est nécessaire entre le dispositif d'analyse 10 et ces équipements. La liaison hertzienne est assurée par un émetteur-récepteur radiofréquence 80 relié à un terminal 82 émetteur-récepteur universel synchrone/asynchrone de données du processeur de données 40 (USART). L'émetteur récepteur radiofréquence est alimenté en énergie par le générateur électrique 56 ou par la batterie.

Une autre sortie de données 84 du processeur de données 40 est utilisée pour commander un dispositif d'éclairage 86 dont la couleur change en fonction des données d'analyse de la qualité sanitaire de l'eau. Le dispositif d'éclairage 86 comprend des diodes électroluminescentes de trois couleurs différentes qui, par synthèse de couleur, permettent de produire différentes couleurs résultantes indicatrices de la qualité de l'eau. Le dispositif d'éclairage 86 peut également être utilisé pour afficher des défauts, des alertes relatives à la qualité sanitaire de l'eau et/ou au fonctionnement interne du dispositif d'analyse.

Dans l'exemple de réalisation de la figure 1, le dispositif d'éclairage 86 est disposé à l'intérieur du boîtier étanche 14 et la lumière émise est visible à travers une paroi transparente ou translucide du boîtier étanche 14. Toutefois, le dispositif d'éclairage 86 peut également être extérieur au boîtier étanche 14 et piloté à distance par voie hertzienne ou encore être disposé de sorte à ce que la lumière émise soit visible à travers la chambre d'analyse 12.

A cet effet, la chambre d'analyse 12 est notamment réalisée en une matière transparente, matière plastique transparente notamment, pour permettre le contrôle visuel de la turbidité de l'eau. En effet, le dispositif d'éclairage 86 logé dans le boîtier étanche 14 illumine la chambre d'analyse 12 au travers de la cloison 16, également transparente, et change la couleur de l'eau présente dans la chambre d'analyse 12 en fonction des mesures effectuées et/ou de l'état du dispositif d'analyse 10.

La référence 88 indique une mémoire programmable associée au processeur de données 40. Elle permet de programmer différentes tâches d'analyse ou de commande du processeur de données 40. Elle permet également de stocker des valeurs ou plages de consigne pour les paramètres de l'eau susceptibles d'être analysés.

La mémoire, les convertisseurs analogique-numériques, l'émetteur-récepteur et le processeur de données peuvent être des composants autonomes ou peuvent être intégrés dans un même boîtier sous la forme d'un microcontrôleur dédié.

La référence 81, indique une interface d'entrée-sortie. En sortie, elle peut être équipée de commutateurs électromécaniques ou de commutateurs statiques à semi-conducteurs, auxquels peut au besoin être branché un équipement d'une piscine. Le branchement s'entend comme un branchement filaire au moyen d'un connecteur étanche adapté.

La référence 81 peut aussi être une entrée permettant le raccordement d'une sonde ou d'un capteur externe capable de délivrer un signal appliqué à l'entrée du processeur de données pour s'ajouter aux « données d'analyse » et être éventuellement transmises par voie hertzienne vers des équipements extérieurs.

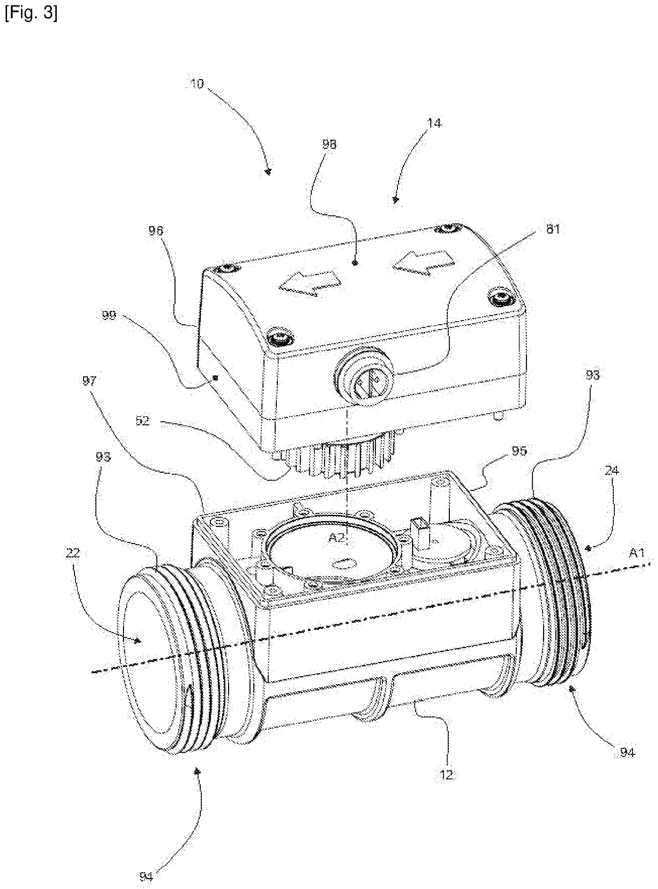

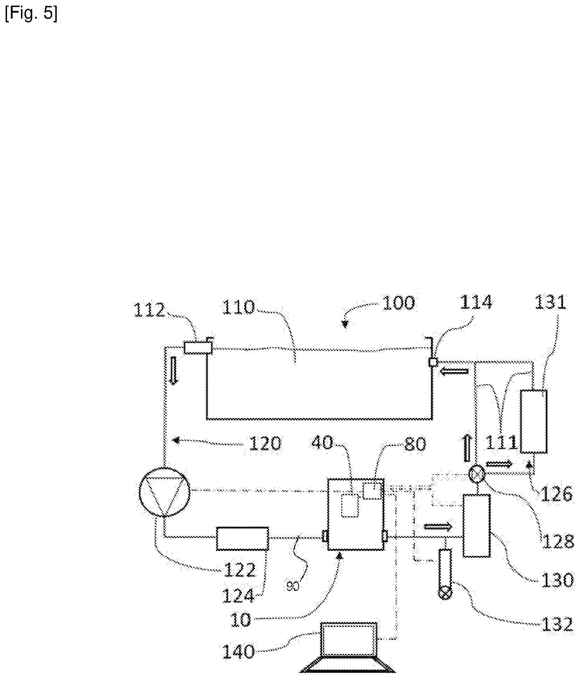

Sur la figure 2, le dispositif d'analyse 10 est installée sur une conduite 90 constitutive d'un circuit de filtration d'eau d'une piscine illustré sur la figure 5. A cet effet, le dispositif d'analyse 10 comprend deux brides annulaires 91 qui sont constitutives d'un dispositif de raccordement 92 du dispositif d'analyse 10 sur la conduite 90. Chaque bride annulaire 91 est par exemple munie de reliefs 93 favorisant une préhension de la bride annulaire 91 lors du raccordement du dispositif d'analyse 10 sur la conduite 90. Un tel raccordement est notamment obtenu à partir d'une rotation sur elle-même de chaque bride annulaire 91 autour de l'axe de révolution A1 pour associer un filetage de la bride annulaire 91 sur un filetage complémentaire 93 ménagé sur la conduite 90 et sur chaque extrémité axiale 94 de la chambre d'analyse 12, tel qu'illustré sur la figure 3.

Sur la figure 3, le boîtier étanche 14 comprend une embase 95 solidaire de la chambre d'analyse 12 et un capot 96 rapportable sur l'embase 95 par l'intermédiaire d'un plan de joint 97. On note que le plan de joint 97 s'étend par exemple à l'intérieur d'un plan orthogonal à l'axe de rotation A2 de l'hélice 52 de la turbine 50.

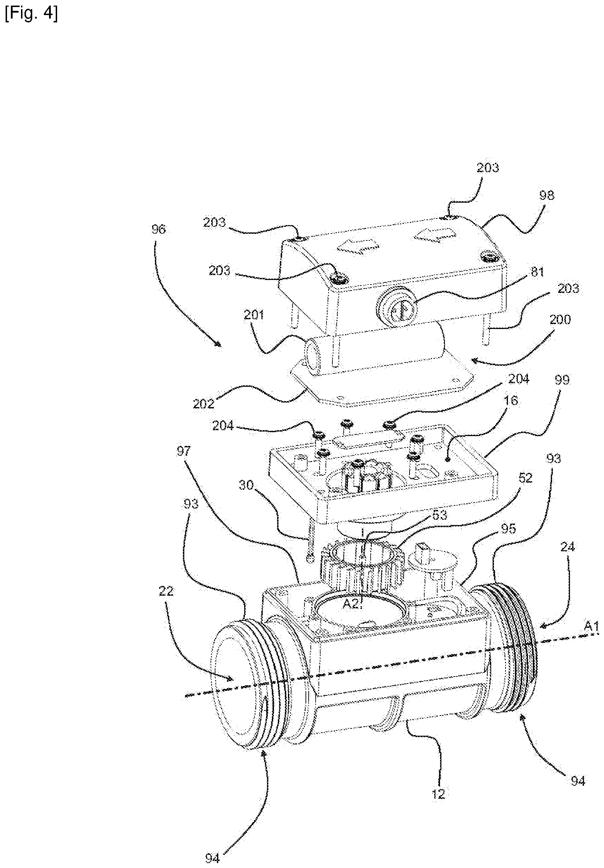

Sur la figure 4, le capot 96 comprend un couvercle 98 et une pièce de liaison 99, la pièce de liaison 99 étant interposée entre le couvercle 98 et l'embase 95. La pièce de liaison 99 est équipée de la cloison étanche 16. La pièce de liaison 99 délimite avec le couvercle 98 un volume interne 200 apte à loger des composants électriques et/ou électroniques 201, notamment disposés sur une carte électronique 202. Le couvercle 98 est rapporté sur la pièce de liaison 99 par l'intermédiaire de premiers moyens de vissage 203 ou analogue. La pièce de liaison 99 est également rapportée sur l'embase par l'intermédiaire de deuxièmes moyens de vissage 204.

La figure 5 montre schématiquement une piscine 100 équipée d'un dispositif d'analyse 10 conforme à l'invention.

La piscine 100 comprend un bassin 110 pourvu d'un écumeur 112 (skimmer) et d'un circuit de filtration 120 reliant l'écumeur 112 à une buse de refoulement 114.

Le circuit de filtration 120 comprend, dans l'ordre depuis l'écumeur 112, une pompe de circulation 122, un filtre 124, le dispositif d'analyse 10 tel que décrit précédemment et une unité de traitement de l'eau 130. Le dispositif d'analyse 10 est placé entre le filtre 124 et la buse de refoulement 114. Les différents organes sont reliés entre eux par des conduites d'eau 111. Les conduites d'eau 111, représentées symboliquement, sont formées de tuyaux et canalisations. La circulation de l'eau est indiquée par de petites flèches au voisinage des conduites d'eau.

L'unité de traitement de l'eau 130 peut comporter, par exemple, une source de rayonnement ultraviolet, et/ou une cellule d'électrolyse.

Par ailleurs, une dérivation 126 du circuit de filtration 120 comprend une vanne 128 et une unité de chauffage 131 pour le réchauffement de l'eau. L'unité de chauffage 131 peut être un échangeur d'une chaudière ou un panneau solaire à circulation, par exemple.

Dans l'exemple de réalisation de la figure 5, la vanne 128 est une vanne motorisée à trois voies, pilotée par le dispositif d'analyse 10. Elle permet de dériver ou non tout ou partie de l'eau du circuit de filtration 120 pour la réchauffer. La proportion de l'eau circulant dans la dérivation est commandée, par exemple, en fonction d'une température de l'eau établie par la sonde de température 32 indiquée sur la figure 1.

Une pompe doseuse 132 est reliée à une conduite du circuit de filtration 120. La pompe doseuse 132 est configurée pour ajouter au besoin à l'eau un additif destiné à corriger sa composition chimique. Plusieurs pompes doseuses peuvent être installées selon le même schéma, pour ajouter plusieurs additifs différents.

La référence 140 désigne un ordinateur portable distant, ou un autre équipement de communication comparable, relié au dispositif d'analyse 10 par l'intermédiaire d'un réseau de communication sans fil, par exemple le réseau Internet, ou un réseau local de courte portée (Wifi, Bluetooth, LPWAN). Les données d'analyse ou d'alerte élaborées par le processeur de données 40 du dispositif d'analyse 10 sont transférées vers l'ordinateur portable 140 ou un équipement de communication comparable. Elles peuvent y être affichées et visualisées au moyen d'une interface graphique dédiée.

Par ailleurs, les différents équipements du circuit de filtration, et en particulier la pompe de circulation 122, la vanne 128, l'unité 130 de traitement de l'eau et la pompe doseuse 132 reçoivent des données de commande du processeur de données 40 du dispositif d'analyse. Ces données sont également transmises sans fil, au moyen de l'émetteur-récepteur radiofréquence 80 du dispositif d'analyse à un récepteur distant. La liaison hertzienne entre le dispositif d'analyse 10 et les différents équipements en communication avec le dispositif d'analyse 10, est représentée symboliquement par des traits mixtes. Par simplification, seuls le processeur de données 40 et l'émetteur récepteur 80 du dispositif d'analyse 10, sont représentés sur la figure 2.

Les données de commande peuvent être transmises directement par le dispositif d'analyse 10 aux différents équipements du circuit de filtration 120. Ceci suppose que chaque équipement possède un émetteur-récepteur de données et d'une interface permettant de piloter son fonctionnement en réponse aux données de commande élaborées par le processeur de données du dispositif d'analyse. Selon une autre possibilité, les données de commande peuvent également être transmises à une interface de pilotage commune à différents équipements. Cette interface peut être disposée à proximité des équipements dans un local technique de la piscine, non représenté. Dans ce dernier cas, les équipements du circuit de filtration peuvent être reliés à l'interface de pilotage commune par voie filaire.

Les données de commande élaborées par le processeur de données 40 peuvent être proportionnelles au flux d'eau dans le circuit de filtration ou peuvent être conditionnées à l'existence d'un écoulement d'eau dans ce circuit. L'existence d'un écoulement d'eau ou l'importance du flux d'eau est mesurée par le signal provenant du générateur électrique de la manière décrite précédemment en référence à la figure 1.

A titre d'illustration, une injection de liquide correcteur de pH dans les conduites d'eau n'est pas souhaitable lorsque l'eau ne circule pas, pour éviter une accumulation de produits potentiellement agressifs pour les parois des conduites d'eau 111.

Aussi lorsque le processeur de données 40 détecte une absence d'écoulement d'eau, caractérisée par une tension de sortie nulle du générateur électrique, une commande d'inhibition du fonctionnement de la pompe doseuse 132 peut être émise.

Selon une variante de réalisation, le dispositif d'analyse d'eau 10 peut être configuré sous la forme d'un insert, pouvant être logé, et en particulier vissé, dans la buse de refoulement 114 de la piscine. Dans ce cas, l'entrée d'eau 22 et la sortie d'eau 24 de la chambre d'analyse 12 du dispositif d'analyse d'eau sont de préférence coaxiales, dans le prolongement l'une de l'autre et centrées sur un axe de la buse de refoulement. L'eau de la buse de refoulement traversant le dispositif d'analyse sert alors à entraîner la turbine du dispositif. Un tel mode de réalisation permet une installation simple du dispositif d'analyse d'eau sur une piscine existante, et, en raison de son caractère autonome, ne nécessite aucun câblage électrique.

Ces dispositions sont telles que le dispositif d'analyse 10 utilise l'énergie hydraulique à la fois pour s'alimenter énergétiquement et accumuler l'énergie nécessaire pour fonctionner de manière autonome plusieurs heures sans circulation d'eau à l'intérieur du circuit de filtration 120. De telles circonstances sont susceptibles de se réaliser lorsque le filtre 124 est encrassé ou bien lorsqu'un objet quelconque obstrue le circuit de filtration 120.

On comprend que la turbine 50 actionne une dynamo 57 ou un alternateur 59 qui convertit la force du mouvement de l'eau dans le circuit de filtration 120 en énergie électrique. La dynamo 57 délivre directement une tension continue tandis que l'alternateur 59 fournit une tension triphasée qui doit être redressée. Dans les deux cas, l'énergie fournie est proportionnelle à la vitesse de circulation de l'eau et donc au débit d'eau dans le circuit de filtration 120. Outre la production d'énergie, la turbine 50 permet donc aussi de détecter l'absence de débit ou des défauts de filtration (colmatage du filtre, skimmer bouché, pompe encrassé, vanne fermée, etc ...) et de mesurer le débit d'eau traversant le dispositif d'analyse 10.

Le dispositif d'analyse 10 calcule le débit de l'eau en fonction d'une tension électrique produite par le convertisseur de tension 64 par l'intermédiaire de la turbine 50. La tension électrique fournie est proportionnelle à la vitesse de rotation de la turbine 50 et donc au débit d'eau traversant le dispositif d'analyse 10.. Ce dernier est donc apte à mesurer le débit d'eau en surveillant la tension fournie par le convertisseur de tension 64. Ce débit est une donnée cruciale pour le bon fonctionnement de la piscine 110 et la détection ou l'absence de déit permet de connaître si la pompe de circulation 122 est en marche ou à l'arrêt. Cette information est utilisée par le dispositif d'analyse 10 pour sa propre gestion et est transmise à un récepteur distant qui en tient compte pour déclencher par exemple une production de chlore ou bien l'injection d'un liquide correcteur de pH dans le circuit de filtration 120. Il est en effet indispensable de veiller à ce que l'eau circule dans le circuit de filtration 120 pour éviter un risque d'accumulation de produits agressifs dans le circuit de filtration 120.

Outre la détection du débit, il est important d'en faire la mesure afin d'ajuster la vitesse de la pompe de circulation 122 ou de déclencher un lavage du filtre 124. A titre d'exemple, il est généralement admis qu'une vitesse de circulation de l'eau de l'ordre de 1,5 m/s à 2 m/s est idéale pour une bonne filtration. Dans une conduite d'un diamètre de 50 mm, cela correspond à un débit de 14 m³/h ce qui permet de recycler quatre fois par jour un bassin de piscine de 56 m³.

L'encrassement du filtre 124 augmente une résistance à l'écoulement et diminue le débit d'eau dans le circuit de filtration 120. L'information de débit est transmise au récepteur peut conduire à un ajustement de la vitesse de filtration et/ou être comparée à un seuil minimum et déclencher un lavage du filtre 124 ou bien une alerte pour l'utilisateur.

L'énergie électrique fournie par la turbine 50 est utilisée pour charger ula batterie 60 qui permet d'une part de stabiliser la tension fournie au processeur de données 40 et d'assurer l'autonomie de fonctionnement du dispositif d'analyse même en l'absence de circulation d'eau à l'intérieur du circuit de filtration 120. En effet, la filtration peut ne fonctionner que quelques heures par jour et le dispositif d'analyse 10 doit pouvoir transmettre les valeurs mesurées et en particulier la température de l'eau pendant les périodes sans filtration. Ainsi, la batterie 60 a une capacité de stockage suffisante pour assurer le fonctionnement du dispositif d'analyse 10 en l'absence de circulation d'eau pendant environ une journée.

Un circuit de surveillance de la charge de la batterie 60 permet d'ajuster la consommation électrique du dispositif d'analyse 10 en fonction du niveau de charge de la batterie 60. Ainsi, lorsque le niveau de charge de la batterie 60 est inférieur à des seuils prédéterminés le dispositif peut diminuer l'intensité du dispositif d'éclairage 86, réduire la fréquence des mesures et des transmission radio, alerter l'utilisateur par des flashs lumineux très courts, envoyer des notifications à un récepteur du type téléphone portable.

## Revendications

1. Dispositif (10) d'analyse d'eau comprenant :
- une chambre d'analyse (12) munie d'au moins une entrée d'eau (22) et au moins une sortie d'eau (24),
- un boîtier étanche (14) adjacent à la chambre d'analyse (12) et isolé de la chambre d'analyse (12) par l'intermédiaire d'une cloison (16),
- un processeur de données (40) logé dans le boîtier étanche (14),
- au moins une sonde (30, 32, 33, 34, 35, 36, 37), reliée électriquement à une entrée de données (42) du processeur de données (40), et s'étendant dans la chambre d'analyse (12), **caractérisé par**
- une alimentation électrique (50, 56, 60, 62) reliée électriquement à une entrée d'alimentation (46) du processeur de données (40), l'alimentation électrique comprenant un générateur électrique (56) pourvu d'une turbine (50) logée dans la chambre d'analyse (12), le générateur électrique (56) délivrant un signal fonction d'une vitesse de rotation de la turbine (50), et étant relié électriquement à l'entrée de données (42) du processeur de données (40) pour la transmission dudit signal au processeur de données, la turbine (50) étant pourvue d'une hélice (52) mobile en rotation sur elle-même autour d'un axe de rotation (A2) qui est orthogonal à un axe de révolution (A1) de la chambre d'analyse (12), la sonde (30, 32, 33, 34, 35, 36, 37) étant logée à l'intérieur d'un compartiment (23) de la chambre d'analyse (12) qui est interposé radialement entre l'entrée d'eau (22) et la cloison (16), l'hélice (52) étant disposée axialement à l'intérieur de la chambre d'analyse (12) entre le compartiment (23) et la sortie d'eau (24).

2. Dispositif d'analyse selon la revendication 1, dans lequel le générateur électrique (56) comprend l'un parmi un alternateur (59) et une dynamo (57).

3. Dispositif d'analyse selon la revendication 2, dans lequel la turbine (50) est pourvue d'un arbre (53) reliant l'hélice (52) à la dynamo (57) ou l'alternateur (59).

4. Dispositif d'analyse selon la revendication 3, dans lequel l'arbre (53) de la turbine (50) traverse la cloison (16) séparant la chambre d'analyse (12) du boîtier étanche (14).

5. Dispositif d'analyse selon la revendication 3, dans lequel le générateur électrique (56) comporte un inducteur (54) à aimant permanent solidaire de la turbine (50) et un induit (58) logé dans le boîtier étanche (14).

6. Dispositif d'analyse selon l'une quelconque des revendications précédentes, dans lequel le signal fonction de la vitesse de rotation de la turbine (50) est une tension de sortie du générateur électrique (56).

7. Dispositif d'analyse selon l'une quelconque des revendications précédentes, dans lequel l'alimentation électrique (50, 56, 60, 62) comprend en outre un accumulateur d'énergie (60) électrique logé à l'intérieur du boîtier étanche (14).

8. Dispositif d'analyse selon l'une quelconque des revendications précédentes, dans lequel le processeur de données (40) est équipé d'un émetteur récepteur hertzien (80) logé dans le boîtier étanche (14) et configuré pour la transmission d'au moins l'un parmi des données d'analyse, des données de commande et des données d'alerte.

9. Dispositif d'analyse selon l'une quelconque des revendications précédentes, dans lequel le processeur de données (40) est équipé d'une interface entrée-sortie externe (81).

10. Dispositif d'analyse selon l'une quelconque des revendications précédentes, dans lequel la sonde (30) est choisie parmi une sonde de température (32), une sonde de pH (33), une sonde de potentiel d'oxydo-réduction (34), une sonde de salinité (35), une sonde de taux de solides dissous (36), et une sonde de turbidité (37).

11. Piscine (100) comprenant un circuit de filtration (120) pourvu d'une pompe de circulation (122), et d'au moins un filtre (124), **caractérisée en ce que** le circuit de filtration (120) est en outre pourvu d'un dispositif d'analyse (10) selon l'une quelconque des revendications précédentes.

12. Piscine (100) selon la revendication 11, dans lequel le dispositif d'analyse (10) est connecté en aval du filtre (124).

13. Piscine (100) selon l'une quelconque des revendications 11 et 12, dans lequel le dispositif d'analyse (10) est connecté en série avec la pompe de circulation (122) et le filtre (124), l'entrée d'eau (22) et la sortie d'eau (24) de la chambre d'analyse (12) étant reliées à une conduite (111) du circuit de filtration.

14. Piscine (100) selon l'une quelconque des revendications 11 à 13, comprenant en outre au moins une pompe doseuse (132) de liquide correcteur.

## Patentansprüche

1. Vorrichtung (10) zur Analyse von Wasser, umfassend:
- eine Analysekammer (12), die mit mindestens einem Wassereinlass (22) und mindestens einem Wasserauslass (24) versehen ist,
- ein wasserdichtes Gehäuse (14), das an die Analysekammer (12) angrenzt und mittels einer Trennwand (16) von der Analysekammer (12) isoliert ist,
- einen Datenprozessor (40), der im wasserdichten Gehäuse (14) untergebracht ist,
- mindestens eine Sonde (30, 32, 33, 34, 35, 36, 37), die elektrisch mit einem Dateneingang (42) des Datenprozessors (40) verbunden ist, und sich in die Analysekammer (12) erstreckt, **gekennzeichnet durch**:
- eine Stromversorgung (50, 56, 60, 62), die elektrisch mit einem Versorgungseingang (46) des Datenprozessors (40) verbunden ist, wobei die Stromversorgung einen elektrischen Generator (56) umfasst, der mit einer Turbine (50) ausgestattet ist, die in der Analysekammer (12) untergebracht ist, wobei der elektrische Generator (56) ein Funktionssignal einer Drehzahl der Turbine (50) liefert, und elektrisch mit dem Dateneingang (42) des Datenprozessors (40) zur Übertragung des Signals an den Datenprozessor verbunden ist, wobei die Turbine (50) mit einem Propellerrad (52) ausgestattet ist, das um sich selbst drehbar und um eine Umlaufsachse (A2) beweglich angeordnet ist, die orthogonal zu einer Drehachse (A1) der Analysekammer (12) ist, wobei die Sonde (30, 32, 33, 34, 35, 36, 37) im Innern eines Raums (23) der Analysekammer (12) untergebracht ist, der radial zwischen dem Wassereinlass (22) und der Trennwand (16) eingesetzt ist, wobei das Propellerrad (52) axial im Innern der Analysekammer (12) zwischen dem Raum (23) und dem Wasserauslass (24) aufgestellt ist.

2. Analysevorrichtung nach Anspruch 1, wobei der elektrische Generator (56) einen von einem Drehstromgenerator (59) und einem Dynamo (57) umfasst.

3. Analysevorrichtung nach Anspruch 2, wobei die Turbine (50) mit einer Welle (53) ausgestattet ist, die das Propellerrad (52) mit dem Dynamo (57) oder dem Drehstromgenerator (59) verbindet.

4. Analysevorrichtung nach Anspruch 3, wobei die Welle (53) der Turbine (50) durch die Trennwand (16) verläuft, die die Analysekammer (12) vom wasserdichten Gehäuse (14) trennt.

5. Analysevorrichtung nach Anspruch 3, wobei der elektrische Generator (56) einen Induktor (54) mit Permanentmagnet beinhaltet, der fest mit der Turbine (50) verbunden ist, und einen Anker (58), der im wasserdichten Gehäuse (14) untergebracht ist.

6. Analysevorrichtung nach einem der vorhergehenden Ansprüche, wobei das Funktionssignal der Drehzahl der Turbine (50) eine Ausgangsspannung des elektrischen Generators (56) ist.

7. Analysevorrichtung nach einem der vorhergehenden Ansprüche, wobei die Stromversorgung (50, 56, 60, 62) ferner einen elektrischen Energiespeicher (60) umfasst, der im Innern des wasserdichten Gehäuses (14) untergebracht ist.

8. Analysevorrichtung nach einem der vorhergehenden Ansprüche, wobei der Datenprozessor (40) mit einem hertzschen Sendeempfänger (80) ausgerüstet ist, der im wasserdichten Gehäuse (14) untergebracht ist, und konfiguriert ist, um die mindestens einen von den Analysedaten, Steuerdaten und Alarmdaten zu übertragen.

9. Analysevorrichtung nach einem der vorhergehenden Ansprüche, wobei der Datenprozessor (40) mit einer externen Eingabe-Ausgabe-Schnittstelle (81) ausgerüstet ist.

10. Analysevorrichtung nach einem der vorhergehenden Ansprüche, wobei die Sonde (30) aus einer Temperatursonde (32), einer pH-Sonde (33), einer Oxidations-Reduktions-Potential-Sonde (34), einer Salzgehalt-Sonde (35), einer gelösten Feststoff-Sonde (36) und einer Trübungssonde (37) ausgewählt ist.

11. Schwimmbad (100), einen Filterkreislauf (120) umfassend, der mit einer Umwälzpumpe (122) ausgestattet ist, und mindestens einem Filter (124), **dadurch gekennzeichnet, dass** der Filterkreislauf (120) ferner mit einer Analysevorrichtung (10) nach einem der vorhergehenden Ansprüche ausgestattet ist.

12. Schwimmbad (100) nach Anspruch 11, wobei die Analysevorrichtung (10) stromabwärts des Filters (124) angeschlossen ist.

13. Schwimmbad (100) nach einem der Ansprüche 11 und 12, wobei die Analysevorrichtung (10) in Reihe geschaltet ist mit der Umwälzpumpe (122) und dem Filter (124), wobei der Wassereinlass (22) und der Wasserauslass (24) der Analysekammer (12) mit einer Leitung (111) des Filterkreislaufs verbunden sind.

14. Schwimmbad (100) nach einem der Ansprüche 11 bis 13, ferner mindestens eine Dosierpumpe (132) für Korrekturflüssigkeit umfassend.

## Claims

1. A device (10) for analysing water comprising:
- an analysis chamber (12) provided with at least one water inlet (22) and at least one water outlet (24),
- a sealed casing (14) adjacent to the analysis chamber (12) and isolated from the analysis chamber (12) via a partition (16),
- a data processor (40) housed in the sealed casing (14),
- at least one probe (30, 32, 33, 34, 35, 36, 37), electrically connected to a data input (42) of the data processor (40), and extending into the analysis chamber (12), **characterised by**
- a power supply (50, 56, 60, 62) electrically connected to a power input (46) of the data processor (40), the power supply comprising an electric generator (56) provided with a turbine (50) housed in the analysis chamber (12), the electric generator (56) delivering a signal depending on a speed of rotation of the turbine (50), and being electrically connected to the data input (42) of the data processor (40) for transmitting said signal to the data processor, the turbine (50) being provided with a propeller (52) movable in rotation on itself around an axis of rotation (A2) which is orthogonal to an axis of revolution (A1) of the analysis chamber (12), the probe (30, 32, 33, 34, 35, 36, 37) being housed inside a compartment (23) of the analysis chamber (12) which is interposed radially between the water inlet (22) and the partition (16), the propeller (52) being disposed axially inside the analysis chamber (12) between the compartment (23) and the water outlet (24) .

2. The analysis device according to claim 1, wherein the electric generator (56) comprises one of an alternator (59) and a dynamo (57).

3. The analysis device according to claim 2, wherein the turbine (50) is provided with a shaft (53) connecting the propeller (52) to the dynamo (57) or the alternator (59) .

4. The analysis device according to claim 3, wherein the shaft (53) of the turbine (50) passes through the partition (16) separating the analysis chamber (12) from the sealed casing (14).

5. The analysis device according to claim 3, wherein the electric generator (56) includes an inductor (54) with a permanent magnet secured to the turbine (50) and an armature (58) housed in the sealed casing (14).

6. The analysis device according to any one of the preceding claims, wherein the signal depending on the speed of rotation of the turbine (50) is an output voltage of the electric generator (56).

7. The analysis device according to any one of the preceding claims, wherein the power supply (50, 56, 60, 62) further comprises an electrical energy accumulator (60) housed inside the sealed casing (14).

8. The analysis device according to any one of the preceding claims, wherein the data processor (40) is equipped with a wireless transceiver (80) housed in the sealed casing (14) and configured for the transmission of at least one of analysis data, control data and alert data.

9. The analysis device according to any one of the preceding claims, wherein the data processor (40) is equipped with an external input-output interface (81).

10. The analysis device according to any one of the preceding claims, wherein the probe (30) is selected from a temperature probe (32), a pH probe (33), an oxidation-reduction potential probe (34), a salinity probe (35), a dissolved solids rate probe (36), and a turbidity probe (37).

11. A swimming pool (100) comprising a filtration circuit (120) provided with a circulation pump (122), and at least one filter (124), **characterised in that** the filtration circuit (120) is further provided with an analysis device (10) according to any one of the preceding claims.

12. The swimming pool (100) according to claim 11, wherein the analysis device (10) is connected downstream of the filter (124).

13. The swimming pool (100) according to any one of claims 11 and 12, wherein the analysis device (10) is connected in series with the circulation pump (122) and the filter (124), the water inlet (22) and the water outlet (24) of the analysis chamber (12) being connected to a pipe (111) of the filtration circuit.

14. The swimming pool (100) according to any one of claims 11 to 13, further comprising at least one correction fluid dosing pump (132).
